# FASCICULE DE BREVET EUROPEEN

(11) **EP 1 066 275 B1**
(45) Date de publication et mention de la délivrance du brevet: **04.09.2002**
(21) Numéro de dépôt: 99916873.5
(22) Date de dépôt: 20.03.1999
(51) Int. Cl.: C07D 301/12, C07D 301/32

(54) **PROCEDE DE FABRICATION D'UN OXIRANNE**
VERFAHREN ZUR HERSTELLUNG EINES OXIRANES
PROCESS FOR PREPARING AN OXIRANE

(30) Priorité: 24.03.1998 BE 9800232
(43) Date de publication de la demande: 10.01.2001
(73) Titulaire: SOLVAY (Société Anonyme), 1050 Bruxelles (BE)
(72) Inventeur: CATINAT, Jean-Pierre, B-7131 Waudrez (BE); STREBELLE, Michel, B-1150 Bruxelles (BE)
(74) Mandataire: Vande Gucht, Anne
(86) Numéro de dépôt international: EP9901956
(87) Numéro de publication internationale: WO99048883

(56) Documents cités:
- EP-A- 0 230 949

## Description

L'invention concerne un procédé de fabrication d'un oxiranne par réaction entre une oléfine et un composé peroxydé en présence d'un catalyseur à base de zéolite. Elle concerne plus particulièrement un procédé de fabrication de 1,2-époxypropane (ou oxyde de propylène) par réaction entre le propylène et le peroxyde d'hydrogène.

Il est connu de fabriquer de l'oxyde de propylène par époxydation de propylène au moyen de peroxyde d'hydrogène et en présence d'un catalyseur de type TS-1, comme décrit par exemple dans la demande de brevet EP 0 230 949. Ce procédé connu présente l'inconvénient de conduire, dans certaines conditions, à des sélectivités faibles.

L'invention vise à remédier à cet inconvénient en fournissant un procédé de fabrication d'un oxiranne qui présente une sélectivité élevée.

L'invention concerne dès lors un procédé continu de fabrication d'un oxiranne selon lequel on fait réagir dans un réacteur en phase liquide une oléfine avec un composé peroxydé en présence d'un catalyseur à base d'une zéolite et en présence d'un solvant, et on introduit en continu dans le réacteur un composé gazeux à un débit suffisant pour entraîner au moins une partie de l'oxiranne produit que l'on recueille avec le composé gazeux à l'endroit où celui-ci sort du réacteur.

Une des caractéristiques essentielles de l'invention réside dans l'introduction d'un composé gazeux dans le réacteur. En effet, il a été constaté que l'oxiranne réagit dans le milieu réactionnel d'époxydation avec l'eau et le solvant pour former des sous-produits ce qui réduit la sélectivité de la réaction d'époxydation. Il a maintenant été trouvé qu'en introduisant un composé gazeux dans le milieu réactionnel à un débit suffisant pour permettre d'entraîner l'oxiranne produit et de le sortir du réacteur en même temps que le composé gazeux, on diminue le temps de contact entre l'oxiranne produit et le milieu réactionnel d'époxydation. On évite ainsi la formation des sous-produits et on augmente la sélectivité de l'époxydation.

La fonction du composé gazeux est d'entraîner l'oxiranne produit hors du milieu réactionnel pour éviter que l'oxiranne soit trop longtemps en contact avec le milieu réactionnel et ainsi éviter la formation de sous-produits. En d'autres mots, le composé gazeux permet d'éliminer l'oxiranne produit du milieu réactionnel par stripping.

Le composé gazeux utilisé dans le procédé selon l'invention peut être tout composé qui se trouve à l'état gazeux dans les conditions de l'époxydation et qui n'a pas d'influence négative sur la réaction d'époxydation. Il peut être choisi parmi les gaz inertes tel que l'azote.

Une forme de réalisation avantageuse du procédé selon l'invention consiste à introduire, dans le réacteur, l'oléfine à l'état gazeux et en un large excès de manière à ce que l'oléfine gazeuse puisse jouer, partiellement ou complètement, le rôle du composé gazeux, à savoir entraîner l'oxiranne produit et le sortir du réacteur.

Une autre forme de réalisation particulière du procédé selon l'invention consiste à introduire le composé gazeux dans le réacteur à un débit tel qu'il permette non seulement d'entraîner au moins une partie de l'oxiranne produit mais également de faire circuler la phase liquide dans le réacteur, en particulier lorsque celui-ci est un réacteur de type boucle.

Dans le procédé selon l'invention le composé gazeux est généralement introduit dans le réacteur à un débit tel que le rapport du débit du composé gazeux au débit d'alimentation du composé peroxydé soit d'au moins 5, en particulier d'au moins 8, les valeurs d'au moins 10 étant courantes. Le rapport de ces débits est généralement inférieur ou égal à 50, en particulier à 30, les valeurs inférieures ou égales à 20 étant courantes.

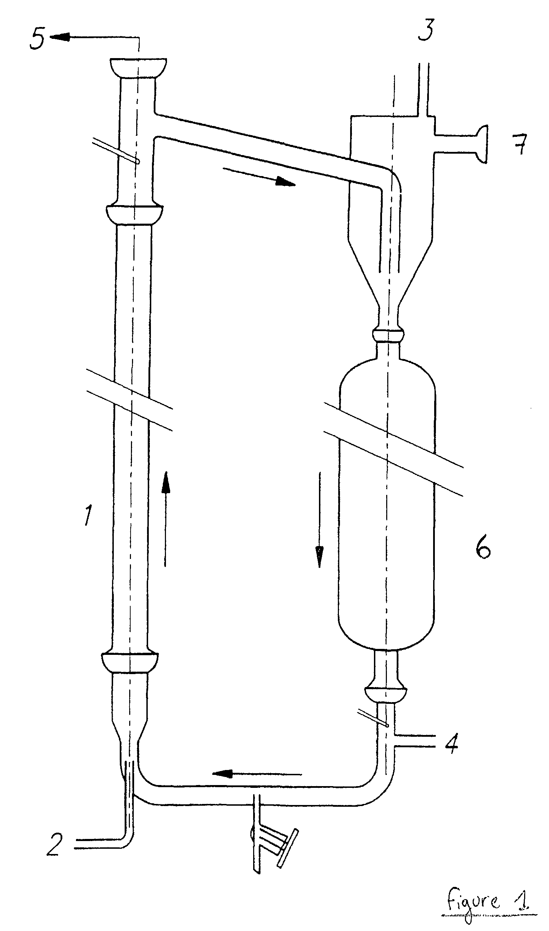

Dans le procédé selon l'invention on peut utiliser tout type de réacteur, en particulier un réacteur de type boucle. Les réacteurs de type boucle à bullosiphon, dans lesquels la circulation du liquide et aussi éventuellement du catalyseur est obtenue par barbotage d'un gaz dans l'une des branches, conviennent bien. Un exemple d'un tel réacteur est schématisé dans la figure 1. Le composé gazeux (de préférence l'oléfine) est introduit dans le pied de la zone de réaction 1 via la canalisation 2. Les autres réactifs (composé peroxydé, solvant, catalyseur, éventuellement un ou plusieurs additifs) sont introduits dans le réacteur via les canalisations 3 et 4. La phase liquide circule dans le réacteur dans le sens des flèches. Le composé gazeux monte dans la zone de réaction 1 et entraîne ainsi l'oxiranne qui y est produit. Un mélange du composé gazeux et d'oxiranne produit sort du réacteur par la canalisation 5. La phase liquide sortant de la tête de la zone de réaction 1 est recyclée dans le pied de la zone de réaction via un échangeur thermique 6. Le débordement de la phase liquide, appauvrie en oxyde de propylene grâce au stripping, est réalisé via la tubulure 7. Peut également être utilisé dans le procédé selon l'invention un réacteur comprenant 2 zones concentriques, la zone centrale assurant la fonction de la zone 1 du réacteur schématisé dans la figure 1 et la zone périphérique assurant la fonction de la zone 6 du réacteur schématisé dans la figure 1.

Dans le procédé selon l'invention il peut s'avérer intéressant de maintenir le pH de la phase liquide lors de la réaction entre l'oléfine et le composé peroxydé à une valeur d'au moins 4,8, en particulier d'au moins 5. Le pH est avantageusement inférieur ou égal à 6,5, en particulier à 6. De bons résultats sont obtenus lorsque le pH est de 4,8 à 6,5, de préférence de 5 à 6. Le pH de la phase liquide lors de la réaction d'époxydation peut être contrôlé par addition d'une base. Cette base peut être choisie parmi les bases solubles dans l'eau. Il peut s'agir de bases fortes. On peut citer à titre d'exemples de bases fortes NaOH et KOH. Il peut également s'agir de bases faibles. Les bases faibles peuvent être inorganiques. On peut citer à titre d'exemples de bases faibles inorganiques NH₄OH, Na₂CO₃, NaHCO₃, Na₂HPO₄, K₂CO₃, Li₂CO₃, KHCO₃, LiHCO₃, K₂HPO₄. Les bases faibles peuvent aussi être organiques. Des bases faibles organiques qui peuvent convenir sont les sels de métaux alcalins ou alcalino-terreux d'acides carboxyliques contenant de préférence de 1 à 10 atomes de carbone. On peut citer à titre d'exemple l'acétate de sodium. Les bases faibles donnent de bons résultats. Les bases faibles organiques sont préférées. L'acétate de sodium convient particulièrement bien.

Les composés peroxydés qui peuvent être utilisés dans le procédé selon l'invention sont les composés peroxydés contenant de l'oxygène actif et capables d'effectuer une époxydation. Le peroxyde d'hydrogène et les composés peroxydés qui peuvent produire du peroxyde d'hydrogène dans les conditions de la réaction d'époxydation conviennent bien. Le peroxyde d'hydrogène est préféré.

Dans le procédé selon l'invention, le composé peroxydé est généralement mis en oeuvre en une quantité d'au moins 1 mol par kg de phase liquide, en particulier d'au moins 1,5 mol par kg de phase liquide. La quantité de composé peroxydé est généralement inférieure à 10 mol par kg de milieu réactionnel; elle est habituellement inférieure ou égale à 5 mol par kg de phase liquide, en particulier inférieure ou égale à 3 mol par kg de phase liquide.

Dans le procédé selon l'invention le composé peroxydé est avantageusement mis en oeuvre sous forme d'une solution aqueuse. En général, la solution aqueuse contient au moins 10 % en poids de composé peroxydé, en particulier au moins 20 % en poids. Elle contient le plus souvent au maximum 70 % en poids de composé peroxydé, en particulier 50 % en poids.

Dans le procédé selon l'invention l'oléfine réagit avec le composé peroxydé en présence du catalyseur et du solvant à une température qui est généralement d'au moins 0 °C, en particulier d'au moins 20 °C. La température est généralement inférieure à 150 °C; elle est habituellement inférieure ou égale à 70 °C, en particulier inférieure ou égale à 40 °C.

Dans le procédé selon l'invention, la réaction entre l'oléfine et le composé peroxydé peut avoir lieu à pression atmosphérique. Elle peut également se dérouler sous pression. Généralement, cette pression n'excède pas 40 bar. Une pression de 20 bar convient bien en pratique.

Les catalyseurs utilisés dans le procédé selon l'invention contiennent une zéolite, à savoir un solide contenant de la silice qui présente une structure cristalline microporeuse. La zéolite est avantageusement exempte d'aluminium. Elle contient de préférence du titane.

La zéolite utilisable dans le procédé selon l'invention peut avoir une structure cristalline de type ZSM-5, ZSM-11, MCM-41 ou de type zéolite bêta. Les zéolites de type ZSM-5 conviennent bien. Celles présentant une bande d'adsorption infrarouge à environ 950-960 cm⁻¹ sont préférées.

Les zéolites qui conviennent particulièrement bien sont les silicalites au titane. Celles répondant à la formule xTiO₂(1-x)SiO₂ dans laquelle x est de 0,0001 à 0,5, de préférence de 0,001 à 0,05 sont performantes. Des matériaux de ce type, connus sous le nom de TS-1 et présentant une structure cristalline de type ZSM-5, donnent des résultats particulièrement favorables.

L'oxiranne qui peut être préparé par le procédé selon l'invention est un composé organique comprenant un groupement répondant à la formule générale :

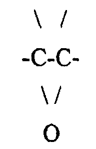

L'oxiranne contient généralement de 2 à 20 atomes de carbone, de préférence de 3 à 10 atomes de carbone. Un oxiranne qui peut être préparé de manière avantageuse par le procédé selon l'invention est le 1,2-époxypropane.

Les oléfines qui conviennent bien dans le procédé selon l'invention contiennent de 3 à 10 atomes de carbone. Le propylène est préféré.

Les solvants utilisables dans le procédé selon l'invention peuvent être les dérivés organiques aliphatiques contenant de 1 à 4 atomes de carbone. On peut citer à titre d'exemple le méthanol.

La teneur initiale en composé peroxydé dans la phase liquide est généralement comprise entre 0,1 et 10 mol/kg. De préférence, elle est comprise entre 1,5 et 3 mol/kg.

### Exemples 1 et 2

De l'oxyde de propylène a été fabriqué dans un réacteur bullosiphon tel que schématisé à la figure 1, par réaction entre du propylène et du peroxyde d'hydrogène 35 % en présence de méthanol et de 5,25 g de catalyseur TS-1, mis en oeuvre sous la forme de billes de 0,5 mm de diamètre.

Les essais ont été réalisés à une température de 35 °C, avec une alimentation continue en peroxyde d'hydrogène à un débit de 0,57 mol/h. La quantité de méthanol mise en oeuvre était de 16 mol/mol d'H₂O₂. Dans l'exemple 1, on a injecté 75 lN/h de propylène (soit 3,3 mol/h). Dans l'exemple 2, on a injecté 250 lN/h de propylène (soit 11,2 mol/h). A ces débits, l'introduction de propylène dans le réacteur a provoqué la mise en circulation du milieu réactionnel liquide et du catalyseur en suspension.

Dans l'exemple 1, on a obtenu une sélectivité en oxyde de propylène de 83 % et un taux de conversion de l' H₂O₂, après 500 heures de réaction, de 76 %.

Dans l'exemple 2, on a obtenu une sélectivité en oxyde de propylène de 90 % et un taux de conversion de l' H₂O₂, après 500 heures de réaction, de 79 %.

(La sélectivité en oxyde de propylène est donnée par le rapport molaire, exprimé en pourcent, entre la quantité d'oxyde de propylène obtenue divisée par la somme de tous les produits organiques formés).

### Exemple 3

De l'oxyde de propylène a été fabriqué dans un réacteur boucle similaire à celui schématisé à la figure 1, par réaction entre du propylène et du peroxyde d'hydrogène 35 % en présence de méthanol et de 8,24 g de catalyseur TS-1 fixé sur un support en nid d'abeilles.

Les essais ont été réalisés à une température de 35 °C, avec une alimentation continue en peroxyde d'hydrogène à un débit de 0,5 mol/h. La quantité de méthanol mise en oeuvre était de 16 mol/mol d'H₂O₂. On a injecté 120 lN/h de propylène et 140 lN/h d'azote.

On a obtenu une sélectivité en oxyde de propylène de 89 % et un taux de conversion de l'H₂O₂, après 1 heure de réaction, de 60 %.

### Exemple 4

De l'oxyde de propylène a été fabriqué dans un réacteur bullosiphon tel que schématisé à la figure 1 par réaction entre du propylène et du peroxyde d'hydrogène 40 % en présence de méthanol et de 5.25 g de catalyseur TS-1, mis en oeuvre sous forme de billes de 0.5 mm de diamètre.

Les essais ont été réalisés à une température de 56 °C, avec une alimentation continue en peroxyde d'hydrogène à un débit de 0.57 mol/h. Le débit de propylène est de 250 lN/h.

Dans un 1er essai, la concentration initiale en H₂O₂ dans le milieu réactionnel (c-à-d en l'absence de réaction) est fixée à 2 mol H₂O₂/kg de phase liquide, ce qui, compte tenu du stripping du CH₃OH, correspond à un rapport CH₃OH/H₂O₂ dans le milieu réactionnel en l'absence de réaction égal à 13 mol/mol.

Dans un 2e essai, on a amené la concentration initiale en H₂O₂ dans le milieu réactionnel (c-à-d en l'absence de réaction) à 6.5 mol H₂O₂/kg de phase liquide, en réduisant simplement le débit de méthanol mis en oeuvre par rapport au 1er essai, de 759 à 375 ml/h. Le rapport CH₃OH/H₂O₂ dans le milieu réactionnel, en l'absence de réaction et compte tenu du stripping du CH₃OH, est voisin dans ces conditions de 2.9 mol/mol.

Dans un 3e essai, le débit de méthanol mis en oeuvre a été réduit à 210 ml/h. Compte tenu du stripping du CH₃OH, la concentration initiale en H₂O₂ passe ainsi à 11.4 mol H₂O₂/kg de phase liquide et le rapport CH₃OH/H₂O₂ dans le milieu réactionnel en l'absence de réaction à 1.3 mol/mol.

Après 6 h d'essai, les taux de conversion de l'H₂O₂ valent respectivement pour les 1er, 2e et 3e essais : 69, 73 et 70 % et les sélectivités en oxyde de propylène sont respectivement égales à 83, 85 et 89 %.

## Revendications

1. Procédé continu de fabrication d'un oxiranne selon lequel on fait réagir dans un réacteur en phase liquide une oléfine avec un composé peroxydé en présence d'un catalyseur à base d'une zéolite et en présence d'un solvant, et on introduit en continu dans le réacteur un composé gazeux à un débit suffisant pour entraîner une partie de l'oxiranne produit que l'on recueille avec le composé gazeux à l'endroit où celui-ci sort du réacteur.

2. Procédé selon la revendication 1, dans lequel l'oléfine est introduite dans le réacteur à l'état gazeux en un large excès et dans lequel l'oléfine joue le rôle du composé gazeux.

3. Procédé selon la revendication 1 ou 2, dans lequel le réacteur est un réacteur boucle.

4. Procédé selon la revendication 3, dans lequel le débit du composé gazeux est suffisant pour faire circuler la phase liquide dans le réacteur boucle.

5. Procédé selon l'une quelconque des revendications précédentes, dans lequel le rapport du débit du composé gazeux au débit d'alimentation du composé peroxydé est supérieur ou égal à 5, de préférence supérieur ou égal à 10.

6. Procédé selon l'une quelconque des revendications précédentes, dans lequel le réacteur est du type boucle à bullosiphon.

7. Procédé selon l'une quelconque des revendications précédentes, dans lequel le pH de la phase liquide est maintenu de 4,8 à 6,5 par l'addition d'une base dans la phase liquide.

8. Procédé selon l'une quelconque des revendications précédentes, dans lequel le composé peroxydé est mis en oeuvre en une quantité de 1 à 10 mol, de préférence de 1,5 à 5 mol, par kg de phase liquide et dans lequel le composé peroxydé est mis en oeuvre sous forme d'une solution aqueuse contenant de 10 à 70 % de composé peroxydé, de préférence de 20 à 50 %.

9. Procédé selon l'une quelconque des revendications précédentes, dans lequel la température à laquelle l'oléfine réagit avec le composé peroxydé en présence du catalyseur et du solvant est de 0 à 150 °C, généralement de 0 à 70 °C, de préférence de 20 à 40 °C.

10. Procédé selon l'une quelconque des revendications précédentes, dans lequel la zéolite est du silicalite au titane, de préférence de type TS-1 présentant une structure cristalline de type ZSM-5.

11. Procédé selon l'une quelconque des revendications précédentes, dans lequel l'oxiranne est le 1,2-époxypropane, l'oléfine est le propylène, le composé peroxydé est le peroxyde d'hydrogène, le solvant est le méthanol et le composé gazeux est du propylène.

## Claims

1. Continuous process for manufacturing an epoxide, according to which an olefin is reacted, in a reactor in the liquid phase, with a peroxide compound in the presence of a zeolite-based catalyst and in the presence of a solvent, and a gaseous compound is introduced continuously into the reactor at a flow rate which is sufficient to entrain some of the epoxide produced, which is recovered with the gaseous compound at the point at which it leaves the reactor.

2. Process according to Claim 1, in which the olefin is introduced into the reactor in gaseous form in a large excess, and in which the olefin acts as the gaseous compound.

3. Process according to Claim 1 or 2, in which the reactor is a loop reactor.

4. Process according to Claim 3, in which the flow rate of the gaseous compound is sufficient to circulate the liquid phase in the loop reactor.

5. Process according to any one of the preceding claims, in which the ratio of the flow rate of the gaseous compound to the flow rate of supply of the peroxide compound is greater than or equal to 5, preferably greater than or equal to 10.

6. Process according to any one of the preceding claims, in which the reactor is of the bubble-siphon loop type.

7. Process according to any one of the preceding claims, in which the pH of the liquid phase is maintained at from 4.8 to 6.5 by addition of a base to the liquid phase.

8. Process according to any one of the preceding claims, in which the peroxide compound is used in an amount of from 1 to 10 mol, preferably from 1.5 to 5 mol, per kg of liquid phase, and in which the peroxide compound is used in the form of an aqueous solution containing from 10 to 70% of peroxide compound, preferably from 20 to 50%.

9. Process according to any one of the preceding claims, in which the temperature at which the olefin reacts with the peroxide compound in the presence of the catalyst and the solvent is from 0 to 150°C, generally from 0 to 70°C, preferably from 20 to 40°C.

10. Process according to any one of the preceding claims, in which the zeolite is titanium silicalite, preferably of TS-1 type having a crystalline structure of ZSM-5 type.

11. Process according to any one of the preceding claims, in which the epoxide is 1,2-epoxypropane, the olefin is propylene, the peroxide compound is hydrogen peroxide, the solvent is methanol and the gaseous compound is propylene.

## Patentansprüche

1. Kontinuierliches Verfahren zur Herstellung eines Oxirans, gemäß dem man in einem Reaktor in flüssiger Phase ein Olefin mit einer Peroxidverbindung in Gegenwart eines Katalysators auf der Basis eines Zeoliths und in Gegenwart eines Lösungsmittels umsetzt und man kontinuierlich in den Reaktor eine gasförmige Verbindung mit einem Durchsatz einführt, der ausreicht, um einen Teil des erzeugten Oxirans mitzureißen, welches man mit der gasförmigen Verbindung an dem Ort, wo diese aus dem Reaktor austritt, gewinnt.

2. Verfahren gemäß Anspruch 1, bei dem das Olefin in den Reaktor in gasförmigem Zustand in einem großen Überschuss eingeführt wird und bei dem das Olefin die Rolle der gasförmigen Verbindung spielt.

3. Verfahren gemäß Anspruch 1 oder 2, bei dem der Reaktor ein Kreislaufreaktor ist.

4. Verfahren gemäß Anspruch 3, bei dem der Durchsatz der gasförmigen Verbindung ausreicht, um die flüssige Phase in dem Kreislaufreaktor zirkulieren zu lassen.

5. Verfahren gemäß einem der vorhergehenden Ansprüche, bei dem das Verhältnis des Durchsatzes der gasförmigen Verbindung zum Einspeisungsdurchsatz der Peroxidverbindung größer oder gleich 5, vorzugsweise größer oder gleich 10 ist.

6. Verfahren gemäß einem der vorhergehenden Ansprüche, bei dem der Reaktor vom Typ Kreislauf mit Blasenheber ist.

7. Verfahren gemäß einem der vorhergehenden Ansprüche, bei dem der pH der flüssigen Phase durch Zugabe einer Base zu der flüssigen Phase auf 4,8 bis 6,5 gehalten wird.

8. Verfahren gemäß einem der vorhergehenden Ansprüche, bei dem die Peroxidverbindung in einer Menge von 1 bis 10 mol, vorzugsweise von 1,5 bis 5 mol pro kg flüssiger Phase eingesetzt wird und bei dem die Peroxidverbindung in Form einer wässrigen Lösung, die 10 bis 70% Peroxidverbindung, vorzugsweise 20 bis 50% enthält, eingesetzt wird.

9. Verfahren gemäß einem der vorhergehenden Ansprüche, bei dem die Temperatur, bei der das Olefin mit der Peroxidverbindung in Gegenwart des Katalysators und des Lösungsmittels reagiert, 0 bis 150°C, im Allgemeinen 0 bis 70°C, vorzugsweise 20 bis 40°C beträgt.

10. Verfahren gemäß einem der vorhergehenden Ansprüche, bei dem der Zeolith ein Titansilikalit vorzugsweise vom Typ TS-1 mit einer Kristallstruktur vom Typ ZSM-5 ist.

11. Verfahren gemäß einem der vorhergehenden Ansprüche, bei dem das Oxiran 1,2-Epoxypropan ist, das Olefin Propylen ist, die Peroxidverbindung Wasserstoffperoxid ist, das Lösungsmittel Methanol ist und die gasförmige Verbindung Propylen ist.
